# EUROPEAN PATENT APPLICATION

(11) **EP 2 799 879 A2**
(43) Date of publication of application: **05.11.2014**
(21) Application number: 14171848.6
(22) Date of filing: 19.08.2010
(51) Int. Cl.: G01N 33/68

(54) **Survival prognostic assay**

(30) Priority: 19.08.2009 GB 0914535; 22.01.2010 GB 201001079
(62) Divisional of application: 10747657.4
(71) Applicant: The Binding Site Group Limited, Birmingham, West Midlands B15 1QT (GB)
(72) Inventor: Mead, Graham, Birmingham West Midlands B15 1QT (GB); Bradwell, Arthur Randall, Birmingham West Midlands B15 1QT (GB)
(74) Representative: Elsy, David

(57) **Abstract**

The invention provides an assay kit for the determination of the total amount of free light chains (FLC) in a serum, whole blood or plasma sample comprising two or more detection agents to detect the total amount of FLC in a sample, the one or more detection agents comprising polyclonal anti-free λ antibodies or fragments thereof and polyclonal anti-free κ antibodies or fragments thereof, wherein the antibodies or fragments are mixed together.

The invention also provides an assay kit for the determination of the total amount of free light chains (FLC) in a sample comprising two or more detection agents to detect the total amount of FLC in a sample, wherein the assay kit is an ELISA, a flow cytometry kit comprising different size beads, a nephelometric or turbidimetric kit.

## Description

The invention relates to a prognostic assay and kit for the determination of the likely survival of a subject or individual.

There are a number of previously identified factors which when measured in a subject, such as a human individual, are indicative of the likely long-term survival of that individual. These include, for example, cholesterol, C-reactive protein (CRP) and cystatin C. Elevated levels of such compounds are associated with diabetes, hypertension and cardio-vascular diseases and renal impairment.

The Applicants have for many years studied free light chains as a way of assaying for a wide-range of monoclonal gammopathies in patients. The use of such free light chains in diagnosis is reviewed in detail in the book "Serum Free Light Chain Analysis, Fifth Edition (2008) A.R. Bradwell et al, ISBN 0704427028".

Antibodies comprise heavy chains and light chains. They usually have a two-fold symmetry and are composed of two identical heavy chains and two identical light chains, each containing variable and constant region domains. The variable domains of each light-chain/heavy-chain pair combine to form an antigen-binding site, so that both chains contribute to the antigen-binding specificity of the antibody molecule. Light chains are of two types, κ and λ and any given antibody molecule is produced with either light chain but never both. There are approximately twice as many κ as λ molecules produced in humans, but this is different in some mammals. Usually the light chains are attached to heavy chains. However, some unattached "free light chains" are detectable in the serum or urine of individuals. Free light chains may be specifically identified by raising antibodies against the surface of the free light chain that is normally hidden by the binding of the light chain to the heavy chain. In free light chains (FLC) this surface is exposed, allowing it to be detected immunologically. Commercially available kits for the detection of κ or λ free light chains include, for example, "Freelite™", manufactured by The Binding Site Limited, Birmingham, United Kingdom. The Applicants have previously identified that measuring the amount of free κ, free λ and/or free κ/free λ ratios, allows the detection of monoclonal gammopathies in patients. It has been used, for example, as an aid in the diagnosis of intact immunoglobulin multiple myeloma (MM), light chain MM, non-secretory MM, AL amyloidosis, light chain deposition disease, smouldering MM, plasmacytoma and MGUS (monoclonal gammopathies of undetermined significance). Detection of FLC has also been used, for example, as an aid to the diagnosis of other B-cell dyscrasia and indeed as an alternative to urinary Bence Jones protein analysis for the diagnosis of monoclonal gammopathies in general.

Conventionally, an increase in one of the λ or κ light chains is looked for. For example, multiple myelomas result from the monoclonal multiplication of a malignant plasma cell, resulting in an increase in a single type of cell producing a single type of immunoglobulin. This results in an increase in the amount of free light chain, either λ or κ, observed within an individual. This increase in concentration may be determined, and usually the ratio of the free κ to free λ is determined and compared with the normal range. This aids in the diagnosis of monoclonal disease. Moreover, the free light chain assays may also be used for the following of treatment of the disease in patients. Prognosis of, for example, patients after treatment for AL amyloidosis may be carried out.

Katzman et al (Clin. Chem. (2002); 48(9): 1437-1944) discuss serum reference intervals and diagnostic ranges for free κ and free λ immunoglobulins in the diagnosis of monoclonal gammopathies. Individuals from 21-90 years of age were studied by immunoassay and compared to results obtained by immunofixation to optimise the immunoassay for the detection of monoclonal free light chains (FLC) in individuals with B-cell dyscrasia.

The amount of κ and λ FLC and the κ/λ ratios were recorded allowing a reference interval to be determined for the detection of B-cell dyscrasias.

The Applicants have now identified that assaying for FLC and especially total FLC can be used to predict long-term survival of individuals, even when the individual is an apparently healthy subject. They have found that FLC concentration is statistically, significantly linked to long-term survival. Moreover, this link appears to be similar or better than the link for existing long-term survival prognostic markers such as cholesterol, creatinine, cystatin C and C-reactive protein.

The concentration of FLC in serum from individuals that are apparently healthy is influenced to some extent by the ability of the individual's kidneys to filter and excrete FLC. In individuals where FLC clearance is restricted, there is an increase in the levels of FLC found in serum. As a consequence, it is now believed that FLC is a good marker of renal function. Because monomeric FLC kappa molecules (25kDa) are of different size to dimeric lambda molecules (50kDa), together they are better markers of glomerular filtration than, for example, cystatin C (10kDa). However, in contrast to cystatin C, production of FLCs during B-cell proliferation are a consequence of many diseases, so serum FLCs will typically not be used as a renal function marker, in isolation. Data indicates that even in patients with chronic kidney disease (CKD), FLC gives the probability of survival, independent of the renal function.

However, markers of B-cell proliferation/activity are important and because B-cells are responsible for making FLCs, this is clinically useful. FLC production is an early indicator of B-cell up-regulation. In this respect it can complement the use of CRP which is a T-cell mediated marker of inflammatory responses.

High FLC concentrations may well be an indication of chronic renal problems, B-cell activation or B-cell proliferation. Hence, an abnormal FLC assay result may be a marker of a variety of disorders that currently require several tests in combination. The converse of this, when the FLC assay results are normal, indicates good renal function and no B-cell activation or proliferation. Hence, FLC concentration has now been recognised as a good indicator of the general health of an individual.

The invention provides a general health screen method comprising detecting an amount of free light chains (FLC) in a sample from a subject, wherein a lower amount is associated with increased survival and/or better general health, and a higher level of FLC indicates the possible presence of an undetected medical problem. The assay may be used, for example, as part of general health screening as part of a subject's general health check. Alternatively it could be used in patients persistently presenting non-specific "general malaise", "under the weather" or "generally unwell" type symptoms. Alternatively, it could be used in patients to distinguish patients who are unwell from psychological causes of feeling unwell compared with difficult to identify physiological causes of symptoms.

The amount of FLC may be compared to a predetermined normal range of FLC to indicate whether the amount of FLC is higher or lower than the normal range.

The FLC may be kappa or lambda FLC. However, preferably the total FLC concentration is measured as detecting kappa FLC or lambda FLC alone may miss, for example abnormally high levels of one or other FLC produced for example monoclonally in the patient.

Total free light chain means the total amount of free kappa plus free lambda light chains in a sample.

Preferably the subject is an apparently healthy subject.

The term "apparently healthy subject" as used herein, is to be understood as referring to a subject with no apparent symptoms of life-threatening diseases or conditions (such as those mentioned below).

Preferably the method provides a method of prognosis of overall survival in a subject. This may be, for example, in a subject not having a diagnosis of or specific symptoms of B-cell associated disease or dyscrasia.

Preferably the apparently healthy subject does not necessarily have symptoms of a B-cell associated disease. The symptoms may include recurrent infections, bone pain and fatigue. Such a B-cell associated disease is preferably not a myeloma, (such as intact immunoglobulin myeloma, light chain myeloma, non-secretory myeloma), an MGUS, AL amyloidosis, Waldenström's macroglobulinaemia, Hodgkin's lymphoma, follicular centre cell lymphoma, chronic lymphocytic leukaemia, mantle cell lymphoma, pre-B cell leukaemia or acute lymphoblastic leukaemia. Moreover, the individual typically does not have reduced bone marrow function. The individual typically does not have an abnormal λ : κ FLC ratio, typically found in many such diseases.

The term "total free light chains" means the amount of κ and λ free light chains in the sample from the subject.

The sample is typically a sample of serum from the subject. However, whole blood, plasma, urine or other samples of tissue or fluids may also potentially be utilised.

Typically the FLC, such as total FLC, is determined by immunoassay, such as ELISA assays or utilising fluorescently labeled beads, such as Luminex™ beads.

ELISA, for example uses antibodies to detect specific antigens. One or more of the antibodies used in the assay may be labeled with an enzyme capable of converting a substrate into a detectable analyte. Such enzymes include horseradish peroxidase, alkaline phosphatase and other enzymes known in the art. Alternatively, other detectable tags or labels may be used instead of, or together with, the enzymes. These include radioisotopes, a wide range of coloured and fluorescent labels known in the art, including fluorescein, Alexa fluor, Oregon Green, BODIPY, rhodamine red, Cascade Blue, Marina Blue, Pacific Blue, Cascade Yellow, gold; and conjugates such as biotin (available from, for example, Invitrogen Ltd, United Kingdom). Dye sols, metallic sols, chemiluminescent labels or coloured latex may also be used. One or more of these labels may be used in the ELISA assays according to the various inventions described herein, or alternatively in the other assays, labeled antibodies or kits described herein.

The construction of ELISA-type assays is itself well known in the art. For example, a "binding antibody" specific for the FLC is immobilised on a substrate. The "binding antibody" may be immobilised onto the substrate by methods which are well known in the art. FLC in the sample are bound by the "binding antibody" which binds the FLC to the substrate via the "binding antibody".

Unbound immunoglobulins may be washed away.

In ELISA assays the presence of bound immunoglobulins may be determined by using a labeled "detecting antibody" specific to a different part of the FLC of interest than the binding antibody.

Flow cytometry may be used to detect the binding of the FLC of interest. This technique is well known in the art for, e.g. cell sorting. However, it can also be used to detect labeled particles, such as beads, and to measure their size. Numerous text books describe flow cytometry, such as Practical Flow Cytometry, 3rd Ed. (1994), H. Shapiro, Alan R. Liss, New York, and Flow Cytometry, First Principles (2nd Ed.) 2001, A.L. Given, Wiley Liss.

One of the binding antibodies, such as the antibody specific for FLC , is bound to a bead, such as a polystyrene or latex bead. The beads are mixed with the sample and the second detecting antibody. The detecting antibody is preferably labeled with a detectable label, which binds the FLC to be detected in the sample. This results in a labeled bead when the FLC to be assayed is present.

Other antibodies specific for other analytes described herein may also be used to allow the detection of those analytes.

Labeled beads may then be detected via flow cytometry. Different labels, such as different fluorescent labels may be used for, for example, the anti- free λ and anti- free κ antibodies. Other antibodies specific for other analytes described herein may also be used in this or other assays described herein to allow the detection of those analytes. This allows the amount of each type of FLC bound to be determined simultaneously or the presence of other analytes to be determined.

Alternatively, or additionally, different sized beads may be used for different antibodies, for example for different marker specific antibodies. Flow cytometry can distinguish between different sized beads and hence can rapidly determine the amount of each FLC or other analyte in a sample.

An alternative method uses the antibodies bound to, for example, fluorescently labeled beads such as commercially available Luminex™ beads. Different beads are used with different antibodies. Different beads are labeled with different fluorophore mixtures, thus allowing different analytes to be determined by the fluorescent wavelength. Luminex beads are available from Luminex Corporation, Austin, Texas, United States of America.

Preferably the assay used is a nephelometric or turbidimetric method. Nephelometric and turbidimetric assays for the detection of λ - or κ - FLC are generally known in the art, but not for total FLC assays. They have the best level of sensitivity for the assay. λ and κ FLC concentrations may be separately determined or a single assay for total FLC arrived at. Such an assay contains anti-κ and anti-λ FLC antibodies typically at a 50:50 ratio.

Antibodies may also be raised against a mixture of free λ and free κ light chains.

The amount of total FLC may be compared to a standard, predetermined value to determine whether the total amount is higher or lower than a normal value.

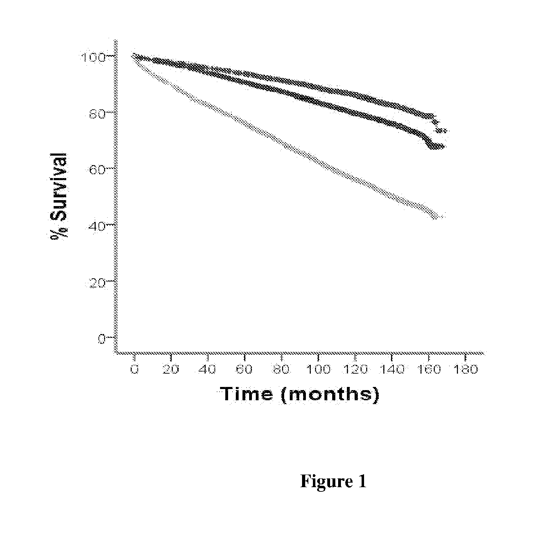

As discussed in detail below, the Applicants have identified that higher than normal concentrations of serum FLC are associated with a significant increase in the likelihood of reduced survival. More so than, for example, for people with lower serum FLC levels. From the data presented herein, normal levels of serum FLC are typically approximately 15-35 mg/L or 20-35 mg/L, 26-35 mg/l, or preferably 25-32, or 24-33 mg/l total FLC. Above 33, for example, above 34, above 36, above 40, above 42, above 48, above 50, above 52, above 55, above 70 mg/l total serum FLC indicates an increase in the likelihood of an earlier death. 33 mg/ml or less than 30, less than 28, less than 26 or less than 25 mg/l in the serum of the individual is associated with a significant increase in the survival of the subject. The increase in probability of survival may be over a period of 2, 4, 6, 8, 10, 12, 14, 16 or more years. The Applicants have identified that the association of lower FLC concentrations with the probability of increased survival is significant over a period of over 160 months. See for example Figure 1.

Historically, assay kits have been produced for measurement of kappa and lambda FLC separately, to allow the calculation of a ratio. They have been conventionally used in individuals already exhibiting disease symptoms.

Preferably the assay is capable of determining FLC, for example total FLC, in the sample for example from approximately 1 mg/L to 100 mg/L, or 1 mg/L - 80 mg/L. This is expected to detect the serum FLC concentrations in the vast majority of individuals without the requirement for re-assaying samples at a different dilution.

The new emphasis of looking at lower concentrations means that preferably the diagnostic assay is capable of detecting FLC below 30 mg/L, more preferably below 20 or below 10 mg/L, below 4 mg/L or 1 mg/L serum FLC.

Preferably the method comprises detecting the amount of total free light chain in the sample utilising an immunoassay, for example, by utilising a mixture of anti-free κ light chain and anti-free λ light chain antibodies or fragments thereof. Such antibodies may be in a ratio of 50:50 anti-κ: anti-λ antibodies. Antibodies, or fragments, bound to FLC may be detected directly by using labelled antibodies or fragments, or indirectly using labelled antibodies against the anti-free λ or anti-free κ antibodies.

The antibodies may be polyclonal or monoclonal. Polyclonal may be used because they allow for some variability between light chains of the same type to be detected as they are raised against different parts of the same chain. The production of polyclonal antibodies is described, for example in WO97/17372.

Preferably, the amount of serum FLC, such as total FLC, identified, and found to be significant to show an increased likelihood of overall survival, is below 33, less than 32, less than 31, less than 30 less than 28, less than 26, less than 25 or less than 24 mg/ml, less than 20 mg/L or less than 15 mg/L.

Conversely, a level above 33 mg/L, especially more than 34, 36, 38, 40, 50, 55, 60 or, above 70 mg/ml is considered to show that the subject has an increased likelihood of overall death. The method may be used to show a worse prognosis in apparently healthy subjects. Or to highlight the probable presence of an undetected medical problem, justifying further investigation.

One or more additional markers may also be tested in the sample. These include cholesterol, creatinine (a marker of renal function), cystatin C or CRP. The use of such assays in general is known in the art. The use of an additional marker is expected to provide further data and improve the accuracy of the prognosis or aid in the diagnosis of an underlying disease/medical problem.

Assay kits for total FLC, for example for use in the methods of the invention are also provided. The kits may detect the amount total FLC in a sample. Such assay kits may be adapted to detect an amount of total free light chain (FLC) in a sample below 25 mg/L, most preferably, below 20 mg/L or about, 10 mg/L, below 5 mg/L or 4 mg/L. The assay kit may be, for example, a nephelometric assay kit. Preferably the kit is an immunoassay kit comprising one or more antibodies against FLC. Typically the kit comprises a mixture of anti-κ and anti-λ FLC antibodies. Typically a mixture of 50:50 anti-free κ and anti-free λ antibodies are used. The kit may be adapted to detect an amount of 1 - 100 mg/L, or preferably 1 - 80 mg/L total free light chain in a sample.

Fragment of antibodies, such as (Fab)₂ or Fab antibodies, which are capable of binding FLC may also be used.

The antibodies or fragments may be labelled, for example with a label as described above. Labelled anti-immunoglobulin binding antibodies or fragments thereof may be provided to detect anti-free λ or anti-free κ bound to FLC.

Kits may form part of a larger test assay kit comprising components for testing other markers, such as cholesterol, creatinine etc., as described above. Antibodies for such markers may be provided

The kit may comprise calibrator fluids to allow the assay to be calibrated at the ranges indicated. The calibrator fluids preferably contain predetermined concentrations of FLC, for example below 25 mg/ml, below 20 mg/ml, below 10 mg/ml, below 5 mg/L or to 1 mg/L. The kit may also be adapted by optimising the amount of antibody and "blocking" protein coated onto latex particles and, for example, by optimising concentrations of supplementary reagents such as polyethylene glycol (PEG) concentrations.

The kit may comprise, for example, a plurality of standard controls for the FLC or indeed other compounds such as cholesterol, creatinine, cystatin C or CRP (c-reactive protein), which may be assayed. The standard controls may be used to validate a standard curve for the concentrations of the FLC or other components to be produced. Such standard controls confirm that the previously calibrated standard curves are valid for the reagents and conditions being used. They are typically used at substantially the same time as the assays of samples from subjects. The standards may comprise one or more standards below 20 mg/L for FLC, more preferably below 15 mg/L, below approximately 10 mg/L or below 5 mg/L, in order to allow the assay to detect the lower concentrations of free light chain.

The assay kit may be a nephelometric or turbidimetric kit. It may be an ELISA, flow cytometry, fluorescent, chemiluminescent or bead-type assay or dipstick. Such assays are generally known in the art.

The assay kit may also comprise instructions to be used in the method according to the invention. The instructions may comprise an indication of the concentration of total free light chain considered to be a normal value, below which, or indeed above which, shows an indication of either increased or decreased probability of survival of the individual. Such concentrations may be as defined above.

The invention will now be described by way of example only, with reference to the following figures:
The invention also provides:
   1. An assay kit for the determination of the total amount of free light chains (FLC) in a sample comprising one or more detection agents to detect the total amount of FLC in a sample.
   2. An assay kit according to 1, comprising anti-free λ and anti-free κ antibodies or fragments thereof.
   3. An assay kit according to 2, wherein the antibodies or fragments are mixed together.
   4. An assay kit according to 2 or 3, wherein the antibodies or fragments are bound to particles, such as latex particles.
   5. An assay kit according to any preceding claim for use in a general health screening method for detecting total FLC in a sample from a subject.
   6. An assay kit for use in a method according to 1 to 5 adapted to detect an amount of total free light chain (FLC) in a sample below 25 mg/L.
   7. An assay kit according to 6 adapted to detect an amount of total FLC of approximately 1 mg/L.
   8. An assay kit according to 1 to 7 adapted to detect an amount of total FLC of 1mg to 80mg/L.
   9. An assay kit according to 1 to 8 which is a nephelometric or turbidimetric kit.
   10. An assay kit according to 1 to 9 additionally comprising one or more components to measure the concentration of one or more of cholesterol, creatinine, cystatin C and CRP in a sample.
   11. An assay kit according to 1 to 10, comprising a plurality of standards for the FLC or components to allow a standard curve for the concentrations of the FLC or components to be produced.
   12. An assay kit according to 11, wherein the standards comprise one or more standards for determining FLC concentration below 25 mg/L.
   13. A general health screen method comprising detecting an amount of free light chains (FLC) in a sample from a subject, wherein a lower amount of FLC is associated with increased survival and/or better general health of the subject, and a higher level of FLC indicates the possible presence of an undetected medical problem.
   14. A method according to 13, wherein the subject does not have apparent symptoms of a life threatening disease, such as a B-cell associated disease or dyscrasia.
   15. A method according to 13 or 14, wherein the amount of free light chains is the amount of total free light chains in the sample.
   16. A method according to 13 to 15, wherein the FLC is determined in a sample of serum from the subject.
   17. A method according to 13 to 16, wherein the total FLC is determined by immunoassay using anti-free light chain antibodies.
   18. A method according to 17, wherein the antibodies are a mixture of anti-free κ light chain and anti-free λ light chain antibodies.
   19. A method according to 13 to 18, wherein the method comprises detecting the amount of FLC by nephelometry or turbidimetry.
   20. A method according to 13 to 19 comprising detecting the FLC with a diagnostic assay, capable of detecting FLC below 3 mg/L.
   21. A method according to 20, wherein the assay is capable of detecting FLC between approximately 1 mg/L and 80 mg/L.
   22. A method according to 13 to 21, wherein the sample is a serum sample and an amount below 25 mg/L of total FLC indicates an increased likelihood of overall survival in a subject.
   23. A method according to 13 to 22 additionally comprising the step of determining an amount of cholesterol, creatinin, cystatin C or C-reactive protein (CRP) in a sample from the subj ect.
   24. An assay kit according to 1 to 12 additionally comprising instructions to be used in a method according to claims 13 to 23.
   25. An assay kit according to 23 additionally comprising a normal value against which a concentration of FLC obtained using the assay kit, indicates an increased survival of a subj ect.

**Figure 1** shows the probability of survival for a study population divided into tertiles on the basis of their total FLC concentrations. The plot shows from top to bottom, line 1, individuals having a median concentration of approximately 20 mg/l total FLC, line 2, individuals having a median concentration of 28.2 mg/l and line 3 (the lower line), a median concentration of approximately 42 mg/l. There were 16554 people in the survival plot.
**Figure 2** shows the range of total FLC concentrations for the tertiles (expressed as mg/decilitre) shown in the survival plot **Figure 1****.**
**Figure 3** shows further analysis of the data expressed as deciles with the plots in decile order at 150 months, with the first decile the top line through to the tenth decile being the bottom line.
**Figure 4** shows the data with MGUS and/or abnormal FLC patients removed with the plots in decile order at 150 months, with the first decile the top line through to the tenth decile being the bottom line.
**Figure 5** is a comparison between the total FLC concentrations obtained using separate, commercially available, anti-free κ and anti-free λ assay kits, compared to a total FLC assay kit using combined anti-λ and anti-κ free light chain antibodies.
**Figure 6** **(a - h)** shows a comparison of c-reactive protein (CRP) levels and FLC levels in serum from patients showing they do not correlate with each other.

The Mayo Clinic in the United States of American has been monitoring >20,000 residents aged 50 or over (as of January 1^{st}, 1995) in Olmsted County, Minnesota. FLC concentrations were measured, retrospectively, in archived, frozen sera, utilising "Freelite™ assays, for free κ - and λ - light chains, from The Binding Site Group Limited, Birmingham, United Kingdom. The Freelite assays are commercially available nephelometric and turbidimetric assays which are FDA approved as an aid in the diagnosis and monitoring of plasma cell disorders such as multiple myeloma and AL amyloidosis. These disorders typically produce an elevated concentration of either λ or κ FLC and an abnormal FLC ratio.

The intent of the investigators at the Mayo Clinic in measuring FLC in the archived sera was to determine whether there were subjects with abnormal FLC ratios and elevation of the concentration of one or both of the FLC. Subjects with such FLC abnormalities but no evidence of monoclonal, intact immunoglobulin production were then investigated to determine whether they had an increased risk of malignant plasma cell disorders.

However, the Applicants speculated that analysis of the subjects without any evidence of monoclonal immunoglobulin or monoclonal FLC production would show increased survival for those with lower total levels of FLC. The data described above demonstrates that this was true.

Figure 1 shows the survival plots for the population divided into tertiles on the basis of their total serum FLC concentrations. The characteristics of the tertiles, one, two and three (top to bottom) are tabulated below:

| **Tertiles of κ + λ FLC (mg/L)** | | | | | | | |
|---|---|---|---|---|---|---|---|
| **Level** | **Minimum** | **10%ile** | **25%ile** | **Median** | **75%ile** | **90%ile** | **Maximum** |
| 1 | 0.83 | 12.8 | 16.6 | 19.995 | 22.24 | 23.5 | 24.2 |
| 2 | 24.2 | 25 | 26.2 | 28.2 | 30.4 | 32 | 33 |
| 3 | 33 | 34.2 | 36.5 | 41.8 | 52.1 | 72.6 | 2743.4 |

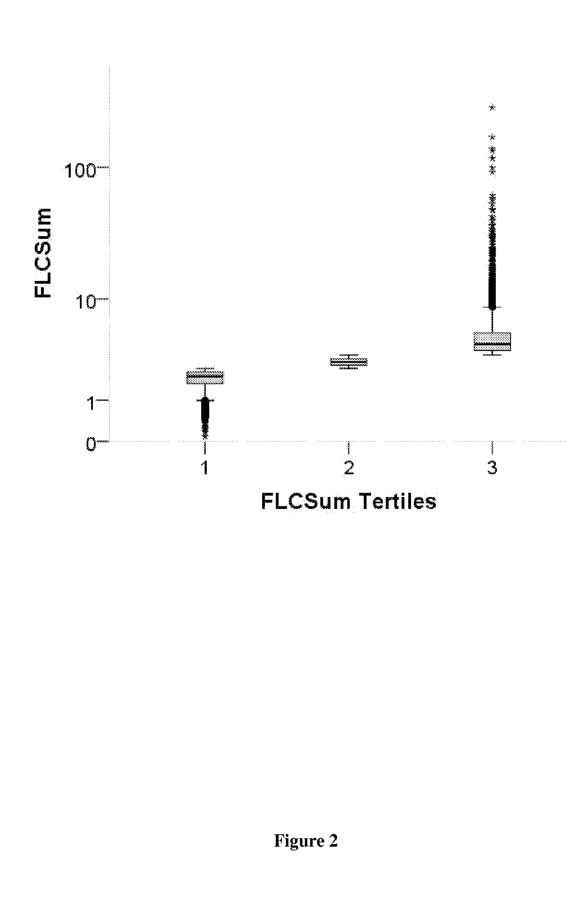

The spread of data is shown in Figure 2.

The data was further analysed to present the data as deciles for all subjects and those without showing signs of MGUS and/or abnormal FLC ratios. The decile levels are shown in the table below:

Decile split summated kappa and lambda free light chain levels (mg/L)

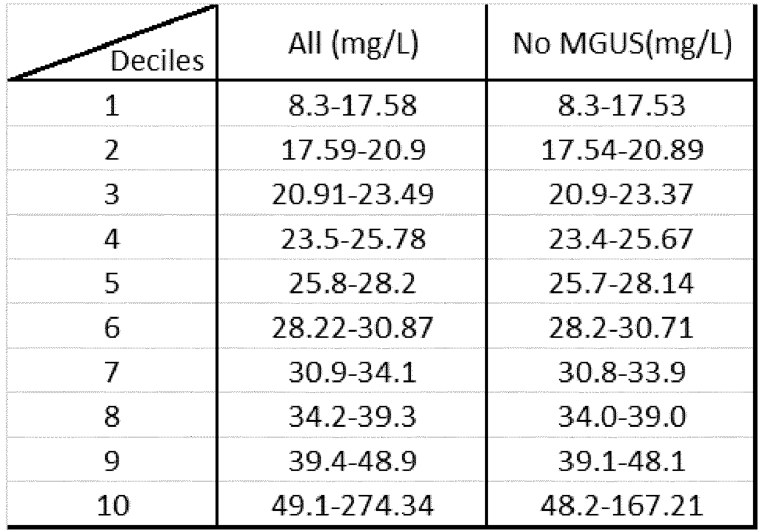

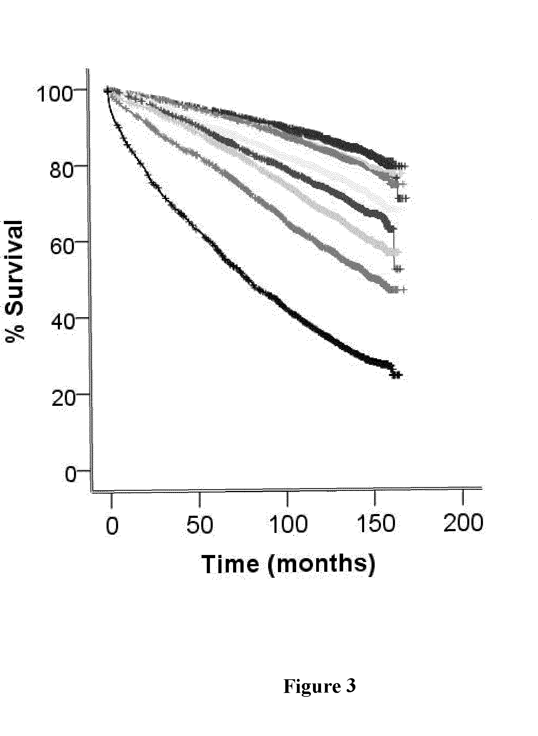

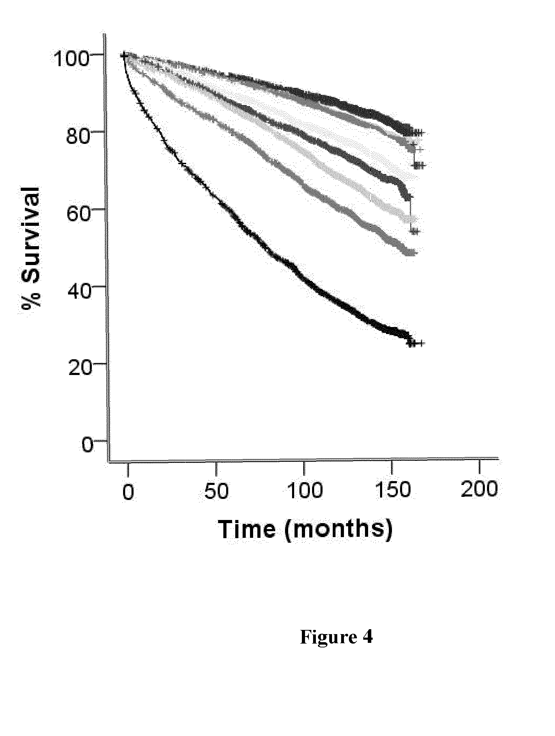

Figure 3 and 4 show the data for all patients and data with MGUS and/ or abnormal FLC ratio patients removed, showing that survival is not significantly due to having MGUS and/or abnormal FLC ratios. The higher the level of FLC, the lower the survival. The lines, top to bottom of the graphs at the 150 month mark, represent deciles 1, 2, 3, 4, 5, 6, 7, 8, 9, and 10 respectively showing good correlation between survival and FLC concentration

### Assay Kit

The method according to the invention may utilise the following assay kit. The assay kit quantifies the total free κ plus free λ light chains present within patient samples, for example, in serum. This may be achieved by coating 100 nm carboxyl modified latex particles with a 50:50 blend of anti-free κ and anti-free λ light chain sheep antibody. In the assay exemplified below, the measuring range for the total free light chains is for 1-80 mg/L. However, other measuring ranges could equally be considered.

Anti-free κ and anti-free λ anti sera are produced using techniques generally known in the art, in this particular case in sheep. The general immunisation process is described in WO 97/17372.

Anti-κ and anti-λ antisera were diluted to equal concentrations using phosphate buffered saline (PBS). Those antibodies were combined to produce antisera comprising 50% anti κ antibody and 50% anti λ antibody.

Antibodies were coated onto carboxyl modified latex at a coat load of 10 mg/lot. This was achieved using standard procedures. See, for example, "Microparticle Reagent Optimization: A laboratory reference manual from the authority on microparticles" Eds: Caryl Griffin, Jim Sutor, Bruce Shull. Copyright Seradyn Inc, 1994 (P/N 0347835(1294).

This reference also provides details of optimising the assay kits using polyethylene glycol (PEG).

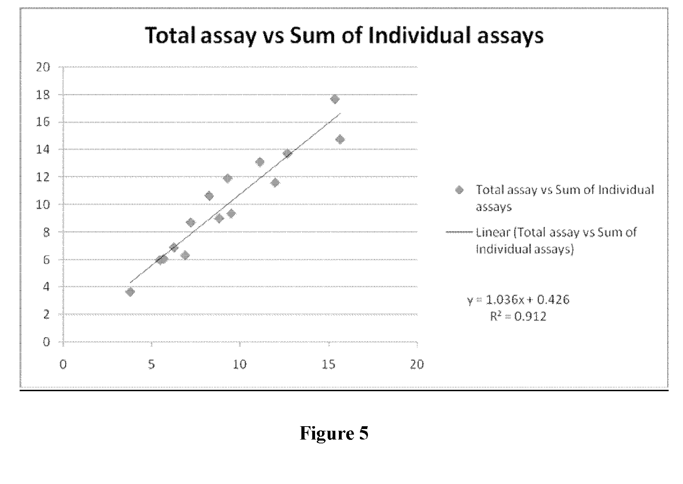

The combined antibodies were compared to results obtained using commercially available κ and λ Freelite™ kits (obtained from the Binding Site Group Limited, Birmingham, United Kingdom). Such Freelite™ kits identify the amount of κ and the amount of λ free light chains in separate assays. The total FLC kits were used to generate curves, which were validated using controlled concentrations. Calibration curves were able to be obtained between 1 and 80 mg/l for total free light chain. In the results table below, results were obtained for κ free light chain (KFLC), λ free light chain (LFLC) and total FLC, using the κ FreeliteTM, λ FreeliteTM and total free light chain assays. These results are shown for 15 different normal serum samples. The results are shown in the table below and in Figure 5 as measured by turbidimetry.

The results indicate that the principle of using a total free light chain assay based on anti-κ and anti-λ free light chain antibody is viable.

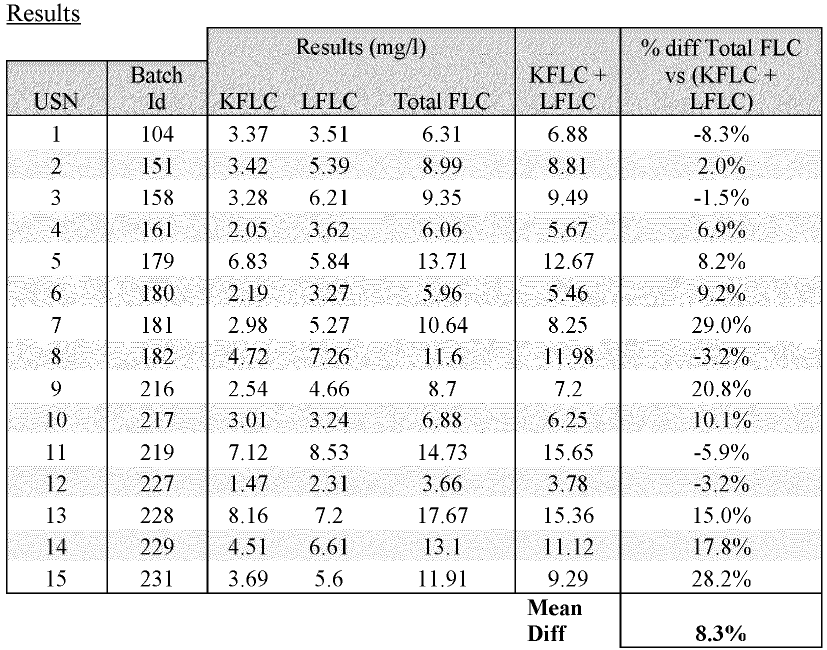

### C-reactive protein (CRP) and FLC concentrations are affected by different factors

### Introduction

C-reactive protein (CRP) is a positive acute phase protein and elevated concentrations are found in serum during inflammatory responses and in response to certain tumours. As immunoglobulin free light chains (FLC) are thought to be produced as a "by-product" of immunoglobulin synthesis, it was possible that there is a degree of correlation between serum concentrations of the two proteins. The purpose of the following study was to determine the concentrations of the two proteins in serial serum samples from patients in an intensive care unit, these patients would, typically, be expected to exhibit significant changes in CRP concentration during their stay in intensive care.

### Patients & Methods

Patients were all undergoing investigations and interventions (within an intensive care unit) for a variety of reasons. Serial blood samples were collected throughout their time in the unit and concentrations of CRP and FLC were measured immunonephelometrically. FLC was determined as above. The CRP was measured using a commercially available kit from Roche (CRPL3 Tima-quant C-Reactive Protein).

### Results

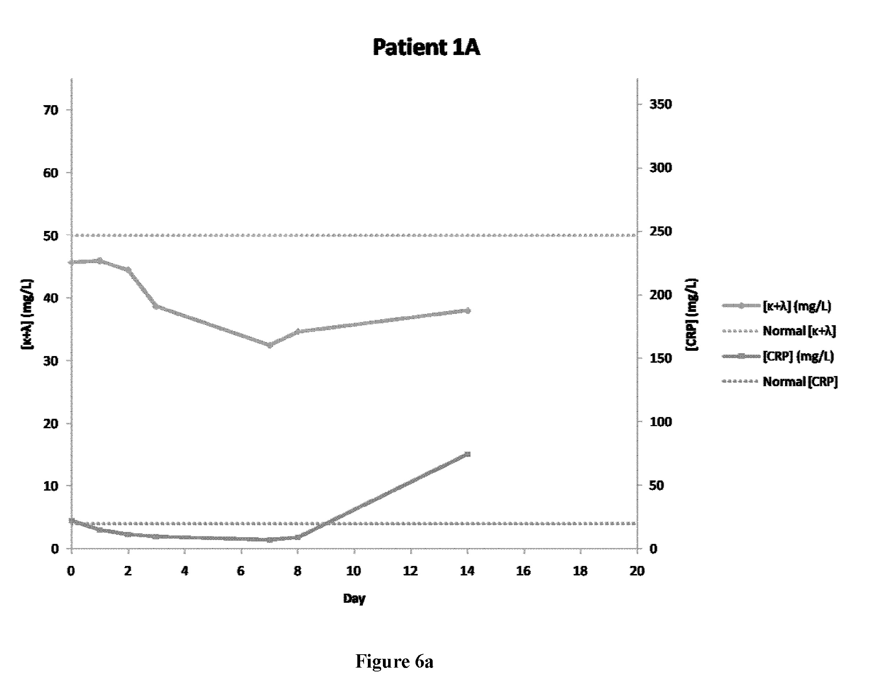

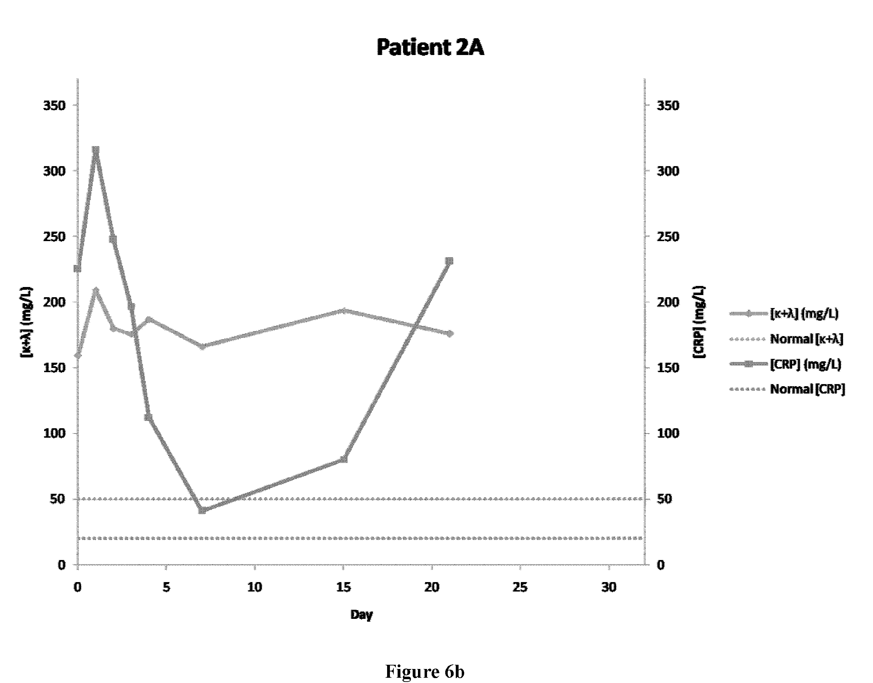

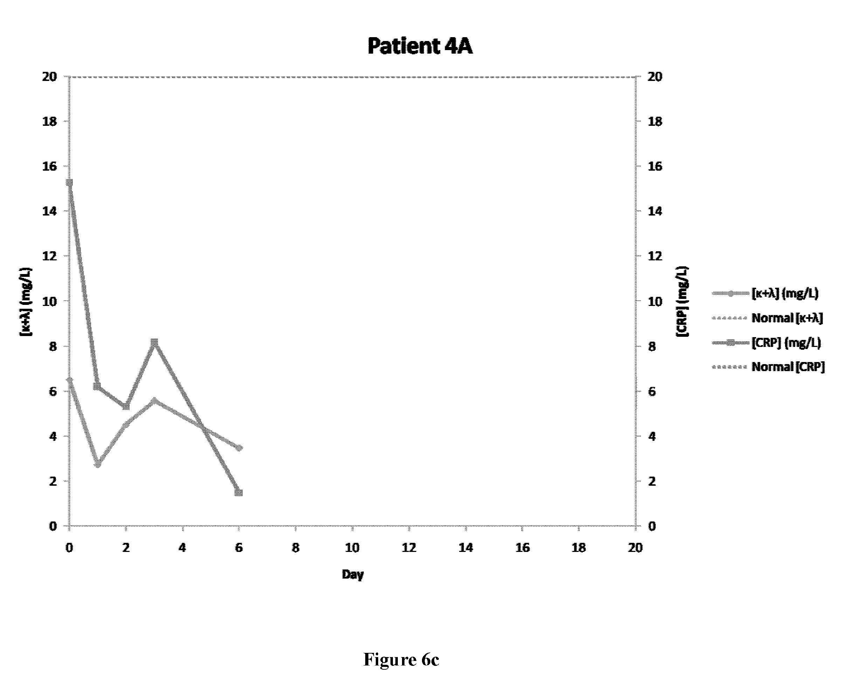

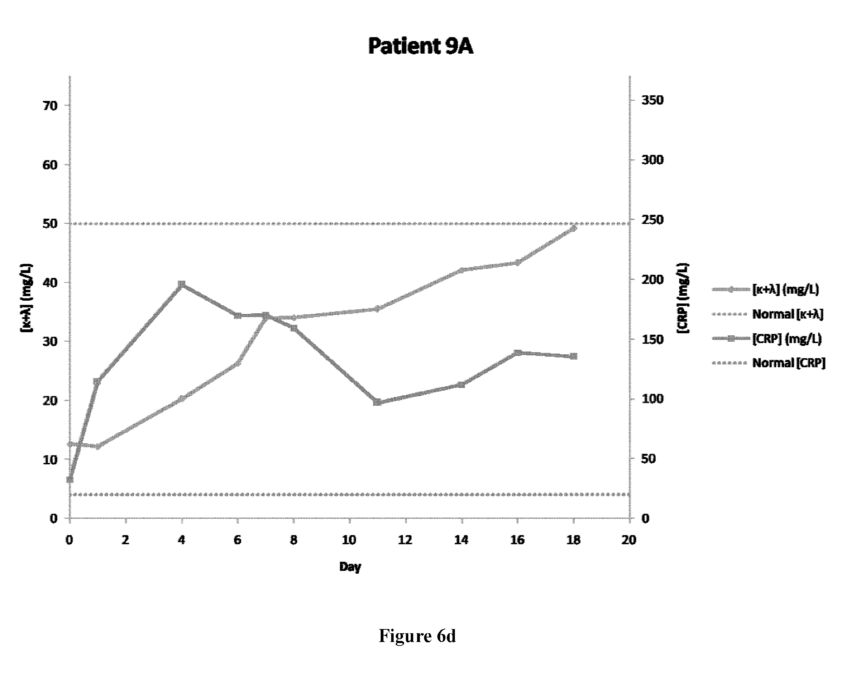

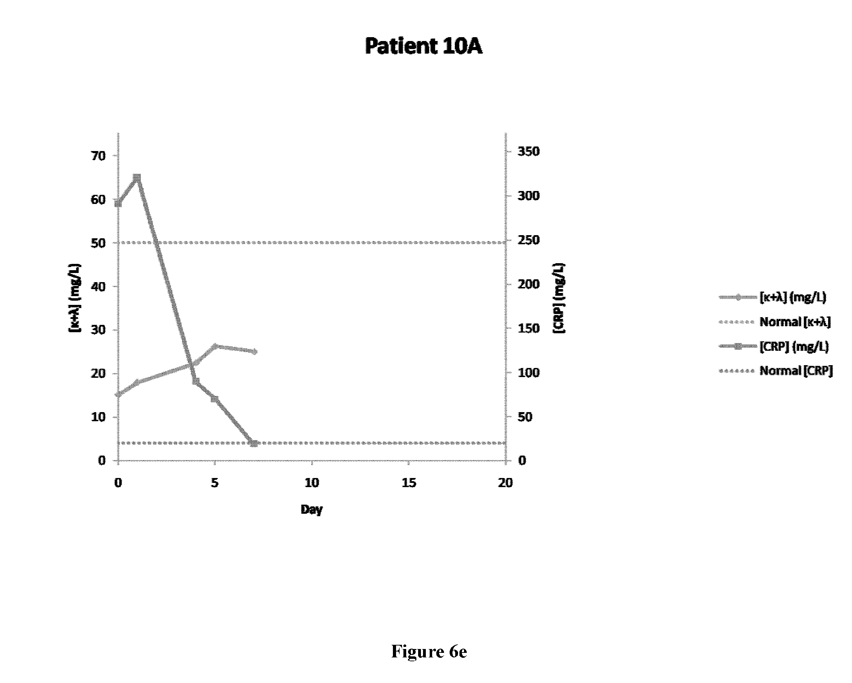

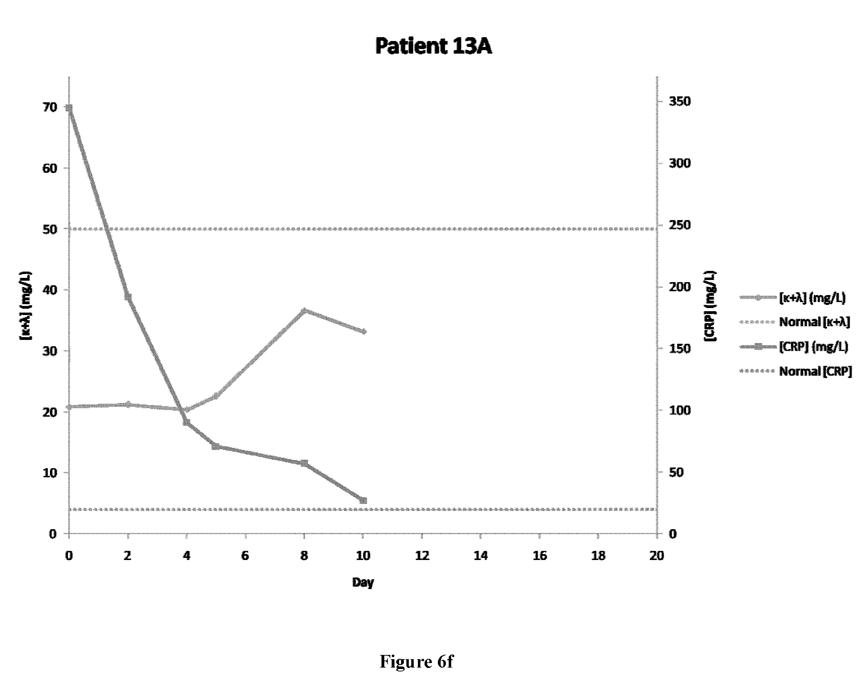

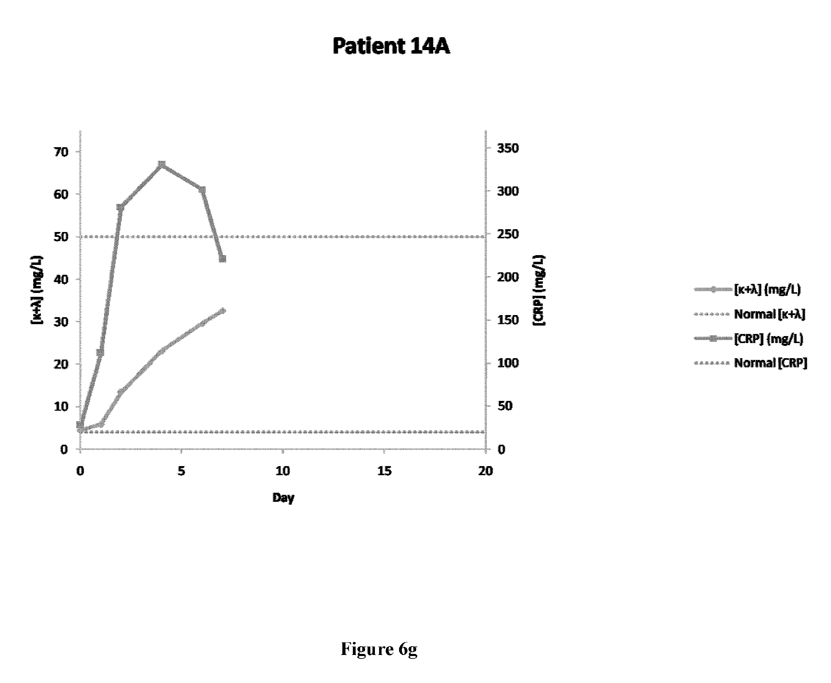

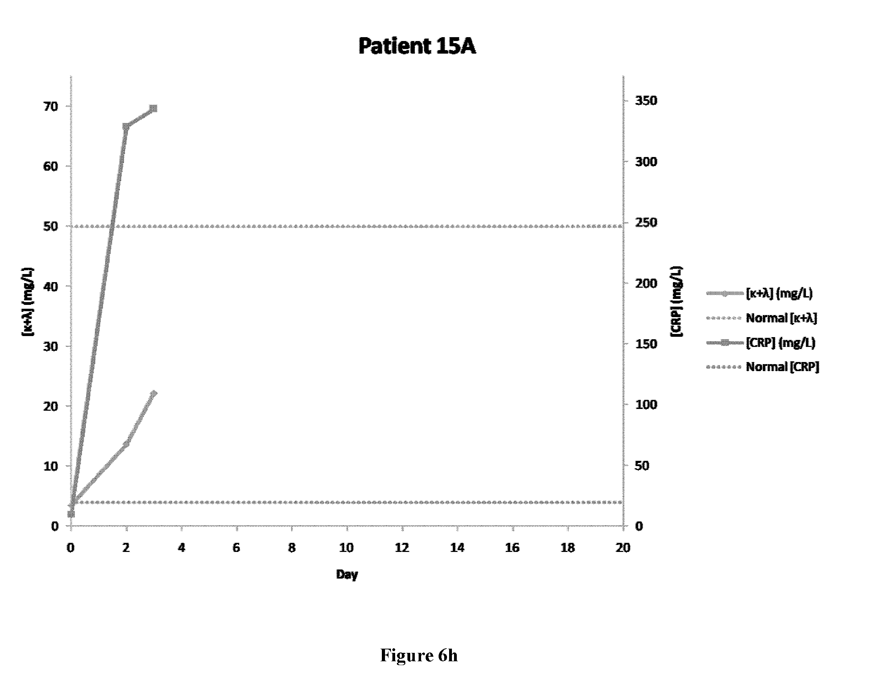

Results are shown on the accompanying graphs (see Figure 6).

Some patients showed reasonably good agreement for the changes in both FLC and CRP concentrations. Other patients showed similar trends for part, but not all, of the monitoring period. In a number of patients the changes in FLC concentration were opposite to those displayed by CRP.

### Conclusions

While some patients showed a degree of similarity between changes in CRP and FLC concentrations, others revealed very different patterns of change. This indicates that different factors are controlling the concentrations of both proteins and they cannot be considered as interchangeable.

### Further Data

The data analysis for survival has also been repeated using different samples from patients elsewhere in the World. A cohort of over 4000 patients from Essen in Germany produced similar results to those described above. Around 500 patients from New Cross Hospital, Wolverhampton, UK who had serum free light chain measurement alongside requests for serum protein electrophoresis were followed up for survival. Similar differences in survival were observed with approximately 98% survival for the first quintile and 50% survival for the fifth quintile after 60 months (data not shown).

1328 patients with chronic kidney disease (CKD) at Queen Elizabeth Hospital, Birmingham, UK were also studied. Of these, 897 had CKD of various causes, 384 had received a kidney transplant and 47 were receiving regular haemodialysis. Survival was studied for patients grouped into different CKD stages based as indicated by serum creatinine concentration with corrections for age, sex and ethnicity (data not shown). The study showed that combined free κ plus free λ concentration still provided an indication of survival risk, independent of CKD stage. Hence FLC concentration and survival is not simply dependent on kidney function but on other factors as well.

## Claims

1. An assay kit for the determination of the total amount of free light chains (FLC) in a serum, whole blood or plasma sample comprising two or more detection agents to detect the total amount of FLC in a sample, the one or more detection agents comprising polyclonal anti-free λ antibodies or fragments thereof and polyclonal anti-free κ antibodies or fragments thereof, wherein the antibodies or fragments are mixed together.

2. An assay kit for the determination of the total amount of free light chains (FLC) in a sample comprising two or more detection agents to detect the total amount of FLC in a sample, wherein the assay kit is an ELISA, a flow cytometry kit comprising different size beads, a nephelometric or turbidimetric kit.

3. An assay kit according to claim 2, wherein the detection agents are a mixture of anti-free lambda specific and anti-free kappa specific antibodies.

4. An assay kit according to any preceding claim, comprising a plurality of standards for the FLC or components to allow a standard curve for the concentrations of the FLC or components to be produced.

5. An assay kit according to claims 1 to 4, comprising a calibrator fluid comprising a predetermined concentration of combined kappa and lambda FLC.

6. An assay kit according to claim 4, wherein the standards comprise one or more standards for determining FLC concentration below 25 mg/L.

7. An assay kit according to any preceding claim, wherein the antibodies or fragments are bound to particles, such as latex particles.

8. An assay kit according to any preceding claim for use in a general health screening method for detecting total FLC in a sample from a subject.

9. An assay kit for use in a method according to any preceding claims, arranged to detect an amount of total free light chain (FLC) in a sample below 25 mg/L, preferably arranged to detect an amount of total FLC of approximately 1 mg/L.

10. An assay kit according to any preceding claim arranged to detect an amount of total FLC of 1mg to 80mg/L.

11. An assay kit according to claims 1 or 3 to 10 which is a nephelometric or turbidimetric kit.

12. An assay kit according to any preceding claim additionally comprising one or more components to measure the concentration of one or more of cholesterol, creatinine, cystatin C and CRP in a sample.

13. An assay kit according to claims 1 to 12 additionally comprising instructions to be used in a general health screen method.

14. An assay kit according to claim 13 additionally comprising a normal value against which a concentration of FLC obtained using the assay kit above which indicates a decreased survival of a subject, or below which indicates an increased survival of a subject.

15. Use of an assay kit to determine the total amount of FLC in a serum, whole blood or plasma sample, the kit comprising two or more detection agents to detect the total amount of FLC in a sample, the one or more detecting agents comprising anti-free λ antibodies or fragments thereof and anti-free κ antibodies or fragments thereof.
